# EUROPÄISCHE PATENTSCHRIFT

(11) **EP 0 455 133 B1**
(45) Veröffentlichungstag und Bekanntmachung des Hinweises auf die Patenterteilung: **05.03.1997**
(21) Anmeldenummer: 91106663.7
(22) Anmeldetag: 25.04.1991
(51) Int. Cl.: C07K 7/18, A61K 38/08, A61K 38/17

(54) **Peptide mit bradykinin-antagonistischer Wirkung**
Peptide antagonists of bradykinin
Peptides ayant une activité antagoniste pour la bradykinine

(30) Priorität: 26.04.1990 DE 4013270
(43) Veröffentlichungstag der Anmeldung: 06.11.1991
(73) Patentinhaber: HOECHST AKTIENGESELLSCHAFT, 65926 Frankfurt am Main (DE)
(72) Erfinder: Breipohl, Gerhard, Dr., W-6000 Frankfurt am Main (DE); Henke, Stephan, Dr., W-6238 Hofheim am Taunus (DE); Knolle, Jochen, Dr., W-6239 Kriftel (DE); Schölkens, Bernward, Prof. Dr., W-6233 Kelkheim/Taunus (DE); Hock, Franz, Dr., W-6110 Dieburg (DE)

(56) Entgegenhaltungen:
- EP-A- 0 370 453
- WO-A-89/01781

## Beschreibung

Die Erfindung betrifft neue Peptide mit bradykinin-antagonistischer Wirkung sowie ein Verfahren zu deren Herstellung.

Bradykinin-antagonistische Peptide werden in WO 86/07263 beschrieben, bei denen u. a. L-Pro in Position 7 des Peptidhormons Bradykinin oder anderer Bradykininanaloga durch eine D-Aminosäure, wie D-Phe, D-Thia, D-Pal, CDF, D-Nal, MDY, D-Phg, D-His, D-Trp, D-Tyr, D-hPhe, D-Val, D-Ala, D-His, D-Ile, D-Leu und DOMT ersetzt ist.

Der Erfindung liegt die Aufgabe zugrunde, neue wirksame Peptide mit bradykinin-antagonistischer Wirkung zu finden.

Diese Aufgabe wird gelöst durch die Peptide der Formel I
H-(D)-Arg-Arg-Pro-Hyp-Gly-E-Ser-(D)-Tic-Oic-Arg-OH (I)
wobei E = Leu, Tbg oder Cha bedeutet
sowie deren physiologisch verträgliche Salze.

Falls nicht anders angegeben, steht die Abkürzung eines Aminosäurerestes ohne einen Stereodeskriptor für den Rest in der L-Form (vgl. Schröder, Lübke, The Peptides, Band I, New York 1965, Seiten XXII-XXIII; Houben-Weyl, Methoden der Organischen Chemie, Band XV/1 und 2, Stuttgart 1974), wie z. B.
Aad, Abu, γAbu, ABz, 2ABz, εAca, Ach, Acp, Adpd, Ahb, Aib, βAib, Ala, βAla, ΔAla, Alg, All, Ama, Amt, Ape, Apm, Apr, Arg, Asn, Asp, Asu, Aze, Azi, Bai, Bph, Can, Cit, Cys, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hIle, hLeu, hLys, hMet, hPhe, hPro, hSer, hThr, hTrp, hTyr, Hyl, Hyp, 3Hyp, Ile, Ise, Iva, Kyn, Lant, Lcn, Leu, Lsg, Lys, βLys, ΔLys, Met, Mim, Min, nArg, Nle, Nva, Oly, Orn, Pan, Pec, Pen, Phe, Phg, Pic, Pro, ΔPro, Pse, Pya, Pyr, Pza, Qin, Ros, Sar, Sec, Sem, Ser, Thi, βThi, Thr, Thy, Thx, Tia, Tle, Tly, Trp, Trta, Tyr, Val.

Als Salze kommen insbesondere Alkali- oder Erdalkalisalze, Salze mit physiologisch verträglichen Aminen und Salze mit anorganischen oder organischen Säuren wie z.B. HCl, HBr, H₂SO₄, H₃PO₄, Maleinsäure, Fumarsäure, Citronensäure, Weinsäure, Essigsäure in Frage.

Beispiele für die Peptide der Formel I sind;
H-(D)-Arg-Arg-Pro-Hyp-Gly-Leu-Ser-(D)-Tic-Oic-Arg-OH
H-(D)-Arg-Arg-Pro-Hyp-Gly-Cha-Ser-(D)-Tic-Oic-Arg-OH
H-(D)-Arg-Arg-Pro-Hyp-Gly-Tbg-Ser-(D)-Tic-Oic-Arg-OH

Die Erfindung betrifft weiterhin ein Verfahren zur Herstellung von Peptiden der Formel I, das dadurch gekennzeichnet ist, daß man
a) ein Fragment mit C-terminaier freier Carboxylgruppe oder dessen aktiviertes Derivat mit einem entsprechenden Fragment mit N-terminaler freier Aminogruppe umsetzt oder
b) das Peptid stufenweise aufbaut,
   in der nach (a) oder (b) erhaltenen Verbindung gegebenenfalls eine oder mehrere zum Schutz anderer Funktionen temporär eingeführte Schutzgruppen abspaltet und die so erhaltenen Verbindungen der Formel I gegebenenfalls in ihr physiologisch verträgliches Salz überführt.

Die Peptide der vorliegenden Erfindung wurden nach allgemein bekannten Methoden der Peptidchemie, siehe z.B. Houben-Weyl, Methoden der organischen Chemie, Band 15/2, bevorzugt mittels Festphasensynthese wie z.B. von B. Merrifield, J. Am. Chem. Soc. 85, 2149 (1963) oder R.C. Sheppard, Int. J. Peptide Protein Res. 21, 118 (1983) beschrieben oder durch äquivalente bekannte Methoden hergestellt. Als α-Aminoschutzgruppe werden Urethanschutzgruppen wie z.B. die tert.-Butyloxycarbonyl(Boc)- oder Fluorenylmethyloxycarbonyl(Fmoc)-Schutzgruppe verwendet. Falls zur Verhinderung von Nebenreaktionen oder für die Synthese spezieller Peptide erforderlich, sind die funktionellen Gruppen in der Seitenkette von Aminosäuren durch geeignete Schutzgruppen (siehe z.B. T.W. Greene, "Protective Groups in Organic Synthesis") zusätzlich geschützt, wobei in erster Linie
Arg(Tos), Arg(Mts), Arg(Mtr), Arg(PMC), Asp(OBzl), Asp(OBut), Cys(4-MeBzl), Cys(Acm), Cys(SBut), Glu(OBzl), Glu(OBut), His(Tos), His(Fmoc), His(Dnp), His(Trt), Lys(Cl-Z), Lys(Boc), Met(O), Ser(Bzl), Ser(But), Thr(Bzl), Thr(But), Trp(Mts), Trp(CHO), Tyr(Br-Z), Tyr(Bzl) oder Tyr(But) eingesetzt werden.

Die Festphasensynthese beginnt am C-terminalen Ende des Peptids mit der Kupplung einer geschützten Aminosäure an ein entsprechendes Harz. Derartige Ausgangsmaterialien können durch Verknüpfung einer geschützten Aminosäure mit einem mit einer Chlormethyl-, Hydroxymethyl-, Benzhydrylamino(BHA)-, Methylbenzhydrylamino(MBHA)-Gruppe modifiziertem Polystyrol- oder Polyacrylamid-Harz über eine Ester- bzw. Amidbindung erhalten werden. Die als Trägermaterial verwendeten Harze sind kommerziell erhältlich. BHA- und MBHA-Harze werden für gewöhnlich verwendet, wenn das synthetisierte Peptid am C-Terminus eine freie Carbamoylgruppe enthalten soll. Falls das Peptid eine sekundäre Amidgruppe am C-terminalen Ende enthalten soll wird ein Chlormethyl- bzw. Hydroxymethyl-Harz verwendet und die Abspaltung mit den entsprechenden Aminen durchgeführt. Will man z.B. das Ethylamid erhalten, kann das Peptid mit Ethylamin vom Harz abgespalten werden, wobei die Abspaltung der Seitenketten-Schutzgruppen nachfolgend durch andere geeignete Reagenzien, erfolgt. Sollen im Peptid die tert.-Butyl-Schutzgruppen der Aminosäure Seitenkette erhalten bleiben, so wird die Synthese mit der Fmoc-Schutzgruppe zur temporären Blockierung der α-Amino-Gruppe der Aminosäure unter Verwendung der z.B. bei R.C. Sheppard, J. Chem. Soc., Chem. Comm. 1982, 587 beschriebenen Methodik durchgeführt, wobei die Guanidino-Funktion des Arginins durch Protonierung mit Pyridinium-Perchlorat geschützt wird und der Schutz der anderen in der Seitenkette funktionalisierten Aminosäuren mit durch katalytische Transferhydrierung (A. Felix et al. J. Org. Chem. 13, 4194 (1978)) oder durch Natrium in flüssigem Ammoniak (W. Roberts, J. Am. Chem. Soc. 76, 6203 (1954)) abspaltbaren Benzylschutzgruppen erfolgt.

Nach Abspaltung der Aminoschutzgruppe der an das Harz gekuppelten Aminosäure mit einem geeigneten Reagenz, wie z.B. Trifluoressigsäure in Methylenchlorid im Falle der Boc-Schutzgruppe oder einer 20 %igen Lösung von Piperidin in Dimethylformamid im Falle der Fmoc-Schutzgruppe, werden die nachfolgenden geschützten Aminosäuren nacheinander in der gewünschten Reihenfolge aufgekuppelt.
Die intermediär entstehenden N-terminal geschützten Peptidharze werden vor der Verknüpfung mit dem nachfolgenden Aminosäurederivat durch die vorbeschriebenen Reagenzien entblockiert.

Als Kupplungsreagenz können alle möglichen in der Peptidsynthese verwendeten Aktivierungs-Reagenzien, siehe z.B. Houben-Weyl, Methoden der organischen Chemie, Band 15/2, verwendet werden, insbesondere aber Carbodiimide wie z.B. N,N'-Dicyclohexylcarbodiimid, N,N'-Diisopropylcarbodiimid oder N-Ethyl-N'-(3-dimethylaminopropyl)carbodiimid. Die Kupplung kann dabei direkt durch Addition von Aminosäurederivat mit dem Aktivierungsreagenz und gegebenenfalls einem die Racemisierung unterdrückenden Zusatz wie z.B. 1-Hydroxybenzotriazol (HOBt) (W. König, R. Geiger, Chem. Ber. 103, 708 (1970)) oder 3-Hydroxy-4-oxo-3,4-dihydrobenzotriazin (HOObt) (W. König, R. Geiger, Chem. Ber. 103, 2054 (1970)) zum Harz durchgeführt werden oder aber die Voraktivierung des Aminosäurederivats als symmetrisches Anhydrid oder HOBt- bzw. HOObt-Ester kann separat erfolgen und die Lösung der aktivierten Spezies in einem geeigneten Lösungsmittel zum kupplungsfähigen Peptidharz gegeben werden.

Die Kupplung bzw. Aktivierung der Aminosäurederivate mit einem der oben genannten Aktivierungsreagenzien kann in Dimethylformamid, N-Methylpyrrolidon oder Methylenchlorid oder einer Mischung aus den genannten Lösungsmitteln durchgeführt werden. Das aktivierte Amlnosäurederivat wird üblicherweise in einem 1,5 bis 4 fachen Überschuß eingesetzt. In Fällen, in denen eine unvollständige Kupplung eintritt, wird die Kupplungsreaktion wiederholt, ohne vorher die für die Kupplung der nächstfolgenden Aminosäure nötige Entblockierung der α-Aminogruppe des Peptidharzes durchzuführen.

Der erfolgreiche Ablauf der Kupplungsreaktion kann mittels der Ninhydrin-Reaktion, wie z.B. von E. Kaiser et al. Anal. Biochem. 34 595 (1970) beschrieben, überprüft werden. Die Synthese kann auch automatisiert z.B. mit einem Peptid-Synthesizer Modell 430A der Fa. Applied Biosystems durchgeführt werden, wobei entweder die vom Gerätehersteller vorgesehenen Syntheseprogramme oder aber auch vom Benutzer selbst erstellte verwendet werden können. Letztere werden insbesondere bei der Verwendung von mit der Fmoc-Gruppe geschützten Aminosäurederivaten eingesetzt.

Nach Synthese der Peptide in der vorgehend beschriebenen Weise kann das Peptid vom Harz mit Reagenzien, wie z.B. flüssigem Fluorwasserstoff (bevorzugt bei den nach der Boc-Methode hergestellten Peptiden) oder Trifluoressigsäure (bevorzugt bei den nach der Fmoc-Methode synthetisierten Peptiden) abgespalten werden. Diese Reagenzien spalten nicht nur das Peptid vom Harz sondern auch die weiteren Seitenkettenschutzgruppen der Aminosäurederivate. Auf diese Weise erhält man außer bei der Verwendung von BHA- und MBHA-Harzen das Peptid in Form der freien Säure. Bei den BHA- bzw. MBHA-Harzen erhält man bei der Spaltung mit Fluorwasserstoff oder Trifluormethansulfonsäure das Peptid als Säureamid. Weitere Verfahren zur Herstellung von Peptidamiden sind in EP-A 287 882 und EP-A 322 348 beschrieben. Hier erfolgt die Abspaltung der Peptidamide vom Harz durch Behandlung mit in der Peptidsynthese üblicherweise verwendeten mittelstarken Säuren (z.B. Trifluoressigsäure) wobei als Kationenfänger Substanzen wie Phenol, Kresol, Thiokresol, Anisol, Thioanisol, Ethandithiol, Dimethylsulfid, Ethylmethylsulfid oder ähnliche in der Festphasensynthese übliche Kationenfänger einzeln oder eine Mischung von zwei oder mehr dieser Hilfsmittel zugesetzt werden. Die Trifluoressigsäure kann dabei auch durch geeignete Lösungsmittel, wie z.B. Methylenchlorid, verdünnt angewendet werden.

Falls die tert.-Butyl bzw. Benzylseitenketten-Schutzgruppen der Peptide erhalten bleiben sollen, wird die Abspaltung des an einem besonders modifizierten Trägerharz synthetisierten Peptides mit 1 % Trifluoressigsäure in Methylenchlorid durchgeführt, wie z.B. bei R.C. Sheppard J. Chem. Soc., Chem. Comm. 1982, 587 beschrieben. Sollen einzelne tert.-Butyl bzw. Benzylseitenketten-Schutzgruppen erhalten bleiben, so verwendet man eine geeignete Kombination der Synthese- und Abspaltmethoden.

Für die Synthese von Peptiden mit einer C-terminalen Carbamoylgruppierung oder einer ω-Amino- bzw. ω-Guanidinoalkylgruppierung wird ebenfalls das von Sheppard beschriebene modifizierte Trägerharz verwendet. Nach der Synthese wird das in der Seitenkette vollgeschützte Peptid vom Harz abgespalten und anschließend in klassischer Lösungssynthese mit dem entsprechenden Amin bzw. ω-Aminoalkylamin oder ω-Guanidinoalkylamin umgesetzt, wobei gegebenenfalls vorhandene weitere funktionelle Gruppen in bekannter Weise temporär geschützt werden können.

Ein weiteres Verfahren zur Herstellung von Peptiden mit einer ω-Aminoalkylgruppierung ist in EP-A 264 802 beschrieben.

Die Peptide der vorliegende Erfindung wurden vorzugsweise unter Verwendung der Festphasentechnik nach zwei generellen Schutzgruppentaktiken synthetisiert:

Die Synthese erfolgte mit einem automatischen Peptidsynthesizer Modell 430 A der Fa. Applied Biosystems unter Verwendung von Boc- bzw. Fmoc-Schutzgruppen zur temporären Blockierung der α-Aminogruppe.

Bei Verwendung der Boc-Schutzgruppe wurden für die Synthese die vom Hersteller des Gerätes vorprogrammierten Synthesezyklen benutzt.

Die Synthese der Peptide mit einer freien Carboxylgruppe am C-terminalen Ende erfolgte an einem mit der entsprechenden Boc-Aminosäure funktionalisiertem 4-(Hydroxymethyl)phenylacetamidomethylpolystyrolharz (R.B. Merrifield, J. Org. Chem. 43, 2845 (1978)) der Fa. Applied Biosystems. Für die Herstellung der Peptidamide wurde ein MBHA-Harz derselben Firma verwendet.
Als Aktivierungsreagenzien dienten N,N'-Dicyclohexylcarbodiimid oder N,N'-Diisopropylcarbodiimid. Die Aktivierung erfolgte als symmetrisches Anhydrid, als HOBt-Ester oder HOObt-Ester in CH₂Cl₂, CH₂Cl₂ - DMF-Gemischen oder NMP. Für die Kupplung wurden 2-4 Äquivalente an aktiviertem Aminosäurederivat eingesetzt. Für Fälle in denen die Kupplung unvollständig verlief, wurde die Reaktion wiederholt.

Bei der Verwendung der Fmoc-Schutzgruppe zum temporären Schutz der α-Aminogruppe wurden für die Synthese mit dem automatischen Peptid-Synthesizer Modell 430A der Fa. Applied Biosystems eigene Syntheseprogramme eingegeben. Die Synthese erfolgte an einem p-Benzyloxybenzylalkohol-Harz (S. Wang, J.Am.Chem.Soc. 95, 1328 (1973)) der Fa. Bachem das nach bekannter Methode (E. Atherton et al. J.C.S.Chem. Comm. 1981, 336) mit der entsprechenden Aminosäure verestert war. Die Aktivierung der Aminosäurederivate als HOBt- oder HOObt-Ester erfolgte direkt in den vom Gerätehersteller gelieferten Aminosäurecartridges durch Zugabe einer Lösung von Diisopropylcarbodiimid in DMF zu der vorher eingewogenen Mischung aus Aminosäurederivat und HOBt oder HOObt. Ebenfalls eingesetzt werden können in Substanz hergestellte Fmoc-Aminosäure-OObt Ester wie sie in EP-A 247 573 beschrieben sind. Die Abspaltung der Fmoc-Schutzgruppe erfolgte mit einer 20 %igen Lösung von Piperidin in DMF im Reaktionsgefäß. Der verwendete Überschuß an reaktivem Aminosäurederivat betrug 1,5 bis 2,5 Äquivalente. Falls die Kupplung nicht vollständig war, wurde sie wie bei der Boc-Methode wiederholt.

Die erfindungsgemäßen Peptide haben einzeln oder in Kombination eine bradykinin-antagonistische Wirkung, die in verschiedenen Modellen getestet werden kann (s. Handbook of Exp. Pharmacol. Vol. 25, Springer Verlag, 1970, S. 53 - 55), so z. B. am isolierten Rattenuterus, am Meerschweinchenileum oder an der isolierten Pulmonalarterie des Meerschweinchens.

Für die Testung der erfindungsgemäßen Peptide an der isolierten Arteria pulmonalis werden Meerschweinchen (Dunkin Hartley) mit einem Gewicht von 400 - 450 g durch Genickschlag getötet.

Der Brustkorb wird geöffnet und die Arteria pulmonalis vorsichtig herauspräpariert. Das umliegende Gewebe wird sorgfältig entfernt und die Arteria pulmonalis in einem Winkel von 45° spiralig aufgeschnitten.

Der Gefäßstreifen von 2,5 cm Länge und 3 - 4 mm Breite wird in einem 10 ml fassenden Organbad, das mit Ringerlösung gefüllt ist, fixiert.

| Zusammensetzung der Lösung in mmol/l | |
|---|---|
| NaCl | 154 |
| KCl | 5,6 |
| CaCl₂ | 1,9 |
| NaHCO₃ | 2,4 |
| Glukose | 5,0 |

Die Lösung wird mit 95 % O₂ und 5 % CO₂ durchperlt und auf 37° C erwärmt. Der pH beträgt 7,4, die Vorlast am Gefäßstreifen beträgt 1,0 g.

Die isometrische Kontraktionsänderungen werden mit einem Hebelvorsatz und einem HF-Modem (Wegmesser) von Hugo Sachs erfaßt und auf einen Kompensationsschreiber (BEC, Goerz Metrawatt SE 460) registriert.

Nach 1 Stunde Äquilibrierung wird mit dem Versuch begonnen. Nachdem die Gefäßstreifen ihre maximale Empfindlichkeit gegenüber 2 x 10⁻⁷ mol/l Bradykinin erreicht haben - Bradykinin führt zu einer Kontraktion der Gefäßstreifen - werden die Peptide in den Dosen 5 x 10⁻⁸ - 1 x 10⁻⁵ mol/l jeweils 10 Minuten einwirken lassen und nach erneuter Gabe von Bradykinin die Abnahme des Effektes von Bradykinin gegenüber der Kontrolle verglichen.

Zur Erkennung eines partialagonistischen Effektes werden die Peptide in den Dosen 1 x 10⁻⁵ - 1 x 10⁻³ mol/l verwendet.

Die aus den Dosiswirkungskurven errechneten IC₅₀-Werte der erfindungsgemäßen Peptide sind in der Tabelle 1 aufgeführt.

**Tabelle 1**

| Verbindung | IC₅₀ _{[M]} |
|---|---|
| H-(D)-Arg-Arg-Pro-Hyp-Gly-Leu-Ser-(D)-Tic-Oic-Arg-OH | 5,9·10⁻⁹ |
| H-(D)-Arg-Arg-Pro-Hyp-Gly-Cha-Ser-(D)-Tic-Oic-Arg-OH | 3,7·10⁻⁸ |
| H-(D)-Arg-Arg-Pro-Hyp-Gly-Tbg-Ser-(D)-Tic-Oic-Arg-OH | 6,0·10⁻⁶ |

Der therapeutische Nutzen der erfindungsgemäßen Peptide umfaßt alle pathologischen Zustände, die durch Bradykinin und Bradykinin verwandte Peptide vermittelt, ausgelöst oder unterstützt werden. Dies beinhaltet u.a. Traumata, wie Wunden, Verbrennungen, Ausschläge, Erytheme, Ödeme, Angina, Arthritis, Asthma, Allergien, Rhinitis, Schock, Entzündungen, niedriger Blutdruck, Schmerz, Juckreiz und veränderte Sperma-Motilität.

Die Erfindung betrifft daher auch die Verwendung von Peptiden der Formel I als Heilmittel und pharmazeutische Präparate, die diese Verbindungen enthalten.

Pharmazeutische Präparate enthalten eine wirksame Menge des Wirkstoffs der Formel I - einzeln oder in Kombination - zusammen mit einem anorganischen oder organischen pharmazeutisch verwendbaren Trägerstoff.

Die Anwendung kann enteral, parenteral - wie z. B. subkutan, i. m. oder i. v. -, sublingual, epikutan, nasal, rektal, intravaginal, intrabukkal oder per Inhalation erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier- oder Dragierverfahren hergestellt.

Für die orale Anwendungsform oder zur Applikation auf die Schleimhäute werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken- und Feuchtgranulat erfolgen. Als ölige Trägerstoffe oder Lösungsmittel kommen beispielsweise pflanzliche oder tierische Öle in Betracht, wie Sonnenblumenöl und Lebertran.

Ein Präparat für die topische Anwendung kann als wäßrige oder ölige Lösung, Lotion, Emulsion oder Gelee, Salbe oder Fettsalbe oder, falls möglich, in Sprayform vorliegen, wobei gegebenenfalls durch Zusatz eines Polymers die Haftung verbessert werden kann.

Für die intranasale Anwendungsform werden die Verbindungen mit den dafür üblichen Zusatzstoffen wie Stabilisatoren oder inerten Verdünnungsmitteln vermischt und durch übliche Methoden in geeignete Darreichungsformen gebracht, wie wäßrige, alkoholische oder ölige Suspensionen oder wäßrige, alkoholische oder ölige Lösungen. Wäßrigen intranasalen Zubereitungen können Chelatbildner, Ethylendiamin-N,N,N',N'-tetraessigsäure, Citronensäure, Weirsäure oder deren Salze zugefügt werden. Die Applikation der Nasallösungen kann mittels Dosierzerstäuber erfolgen oder als Nasaltropfen mit viskositätserhöhendem Anteil bzw. Nasengels oder Nasencremes.

Für die inhalative Anwendung können Vernebler oder Druckgaspackungen unter Verwendung inerter Trägergase benutzt werden.

Zur intravenösen, subkutanen, epikutanen oder intradermalen Applikation werden die aktiven Verbindungen oder deren physiologisch verträgliche Salze, gewünschtenfalls mit den pharmazeutisch üblichen Hilfsstoffen, beispielsweise zur Isotonierung oder pH-Einstellung sowie Lösungsvermittler, Emulgatoren, oder anderen Hilfsstoffen, in Lösung, Suspension oder Emulsion gebracht.

Aufgrund der kurzen Halbwertszeiten einiger der beschriebenen Arzneistoffe in Körperflüssigkeiten ist der Einsatz von injizierbaren Retardzubereitungen sinnvoll. Als Arzneiformen können z. B. ölige Kristallsuspensionen, Mikrokapseln, Rods oder Implantate verwendet werden, wobei die letzteren aus gewebeverträglichen Polymeren, insbesondere bioabbaubaren Polymeren, wie z. B. auf der Basis von Polymilchsäure-Polyglykolsäure-Copolymeren oder Humanalbumin aufgebaut sein können.

Ein geeigneter Dosisbereich für topische und inhalative Anwendungsformen sind Lösungen mit 0,01-5 mg/ml, bei systemischen Applikationsformen sind 0,01-10 mg/kg geeignet.

### Verzeichnis der Abkürzungen:

Die für Aminosäuren verwendeten Abkürzungen entsprechen dem in der Peptidchemie üblichen drei Buchstaben-Code wie er in Europ. J. Biochem. 138, 9 (1984) beschrieben ist. Weitere verwendete Abkürzungen sind nachfolgend aufgelistet.
- Acm: Acetamidomethyl
- ε-Ahx: ε-Aminohexanoyl
- Aoc: cis, endo-2-Azabicyclo[3.3.0]octan-3-S-carbonyl
- Boc: tert.-Butyloxycarbonyl
- But: tert.-Butyl
- Bzl: Benzyl
- CDF: Chlor-(D)-phenylalanyl
- Cha: Cyclohexylalanyl
- Chg: Cyclohexylglycyl
- Cl-Z: 4-Chlor-benzyloxycarbonyl
- DMF: Dimethylformamid
- DOMT: O-Methyl-(D)-Threonyl
- Dnp: 2,4-Dinitrophenyl
- Fmoc: 9-Fluorenylmethyloxycarbonyl
- MDY: O-Methyl-(D)-Tyrosyl
- Me: Methyl
- 4-Mebzl: 4-Methylbenzyl
- Mtr: 4-Methoxy-2,3,6-trimethylphenylsulfonyl
- Mts: Mesitylen-2-sulfonyl
- Nal: 2-Naphthylalanyl
- NMP: N-Methylpyrrolidin
- Npg: Neopentylglycyl
- Oic: cis-endo-Octahydroindol-2-carbonyl
- Opr: Isoxazolidin-3-ylcarbonyl
- Pal: Pyridylalanyl
- Pmc: 2,2,5,7,8-Pentamethylchroman-6-sulfonyl
- Tbg: tert.-Butylglycyl
- TFA: Trifluoressigsäure
- Thia: 2-Thienylalanyl
- Tcs: 4-Methylphenylsulfonyl
- Tic: 1,2,3,4-Tetrahydroisochinolin-3-ylcarbonyl
- Trt: Trityl

Die folgenden Beispiele sollen die bevorzugten Methoden zur Festphasen-Synthese der erfindungsgemäßen Peptide verdeutlichen, ohne daß die Erfindung darauf eingeschränkt wäre.

Man verwendete folgende Aminosäure-Derivate:
Fmoc-Arg(Mtr)-OH, Boc-(D)-Arg-OH, Fmoc-Arg(Pmc)-OH, Fmoc-Hyp-OH, Fmoc-Pro-OObt, Fmoc-Gly-OObt, Fmoc-Phe-OObt, Fmoc-Ser(tBu)-OObt, Fmoc-(D)-Tic-OH, Fmoc-Gln-OH, Fmoc-Aoc-OH, Fmoc-Thia-OH, Fmoc-Opr-OH, Fmoc-(D)-Asn-OH, Fmoc-β-Ala-OH, Fmoc-Oic-OH.

### Beispiel 1:

### H-(D)-Arg-Arg-Pro-Hyp-Gly-Leu-Ser-(D)-Tic-Oic-Arg-OH

wurde mit einem Peptid-Synthesizer Modell 430 A der Fa. Applied Biosystems unter Verwendung der Fmoc-Methode an einem mit Fmoc-Arg(Mtr)-OH veresterten p-Benzyloxybenzylalkohol-Harz der Fa. Novabiochem (Beladung ca. 0,5 mmol/g Harz) stufenweise aufgebaut. Es wurde 1 g des Harzes eingesetzt und die Synthese mit Hilfe eines für die Fmoc-Methode modifizierten Synthese-Programmes durchgeführt.

In die Cartridges des Synthesizers wurden jeweils 1 mmol der Aminosäurederivate mit freier Carboxylgruppe zusammen mit 0,95 mmol HOObt eingewogen. Die Voraktivierung dieser Aminosäuren erfolgte direkt in den Cartridges durch Lösen in 4 ml DMF und Zugabe von 2 ml einer 0,55 mol/l Lösung von Diisopropylcarbodiimid in DMF.
Die HOObt-Ester der anderen Aminosäuren wurden in 6 ml NMP gelöst und dann ebenso wie die in situ voraktivierten Aminosäuren an das vorher mit 20 % Piperidin in DMF entblockierte Harz gekuppelt. Nach beendeter Synthese wurde das Peptid unter gleichzeitiger Entfernung der Seitenkettenschutzgruppen mit Trifluoressigsäure unter Verwendung von Thioanisol und Ethandithiol als Kationenfänger vom Harz abgespalten. Der nach Abziehen der Trifluoressigsäure erhaltene Rückstand wurde mehrfach mit Essigester digeriert und zentrifugiert. Der verbliebene Rückstand wurde an ®Sephadex LH 20 mit 10 %iger Essigsäure chromatographiert. Die das reine Peptid enthaltenden Fraktionen wurden vereinigt und gefriergetrocknet
MS(FAB) : 1264 (M+H)

Die nachfolgenden Beispiele wurden analog Beispiel 1 hergestellt.

### Beispiel 2:

### H-(D)-Arg-Arg-Pro-Hyp-Gly-Cha-Ser-(D)-Tic-Oic-Arg-OH

MS (FAB): 1304 (M+H)

### Beispiel 3:

### H-(D)-Arg-Arg-Pro-Hyp-Gly-Tbg-Ser-(D)-Tic-Oic-Arg-OH

MS (FAB): 1264 (M+H)

## Patentansprüche (Patentansprüche für folgende(n) Vertragsstaat(en): AT, BE, CH, DE, DK, FR, GB, IT, LI, LU, NL, SE)

1. Peptid der Formel I
H-(D)-Arg-Arg-Pro-Hyp-Gly-E-Ser-(D)-Tic-Oic-Arg-OH (I)
wobei
E = Leu, Tbg oder Cha bedeutet
sowie deren physiologisch verträgliche Salze.

2. Verfahren zur Herstellung eines Peptids der Formel I gemäß Anspruch 1, dadurch gekennzeichnet, daß man
a) ein Fragment mit C-terminaler freier Carboxylgruppe oder dessen aktiviertes Derivat mit einem entsprechenden Fragment mit N-terminaler freier Aminogruppe umsetzt oder
b) das Peptid stufenweise aufbaut,
in der nach a) oder b) erhaltenen Verbindung gegebenenfalls eine oder mehrere zum Schutz anderer Funktionen temporär eingeführte Schutzgruppen abspaltet und die so erhaltene Verbindung der Formel I gegebenenfalls in ihr physiologisch verträgliches Salz überführt.

3. Peptid der Formel I gemäß Anspruch 1 zur Anwendung als Heilmittel.

4. Peptid der Formel I gemäß Anspruch 1 zur Behandlung pathologischer Zustände, die durch Bradykinin und Bradykinin verwandte Peptide vermittelt, ausgelöst oder unterstützt wreden.

5. Pharmazeutisches Mittel enthaltend ein Peptid der Formel I gemäß Anspruch 1.

## Patentansprüche (Patentansprüche für folgende(n) Vertragsstaat(en): ES, GR)

1. Verfahren zur Herstellung eines Peptids der Formel I
H-(D)-Arg-Arg-Pro-Hyp-Gly-E-Ser-(D)-Tic-Oic-Arg-OH (I)
wobei
E = Leu, Tbg oder Cha bedeutet
sowie deren physiologisch verträgliche Salze,
dadurch gekennzeichnet, daß man
a) ein Fragment mit C-terminaler freier Carboxylgruppe oder dessen aktiviertes Derivat mit einem entsprechenden Fragment mit N-terminaler freier Aminogruppe umsetzt oder
b) das Peptid stufenweise aufbaut,
in der nach a) oder b) erhaltenen Verbindung gegebenenfalls eine oder mehrere zum Schutz anderer Funktionen temporär eingeführte Schutzgruppen abspaltet
und die so erhaltene Verbindung der Formel I gegebenenfalls in ihr physiologisch verträgliches Salz überführt.

2. Verfahren zur Herstellung eines pharmazeutischen Mittels enthaltend ein Peptid der Formel I gemäß Anspruch 1, dadurch gekennzeichnet, daß man dieses und einen Träger in eine geeignete Darreichungsform bringt.

## Claims (Claims for the following Contracting State(s): AT, BE, CH, DE, DK, FR, GB, IT, LI, LU, NL, SE)

1. A peptide of the formula I
H-(D)-Arg-Arg-Pro-Hyp-Gly-E-Ser-(D)-Tic-Oic-Arg-OH (I)
where
E is Leu, Tbg or Cha
and the physiologically tolerated salts thereof.

2. A process for the preparation of a peptide of the formula I as claimed in claim 1, which comprises
a) reacting a fragment with a C-terminal free carboxyl group or its activated derivative with a corresponding fragment with an N-terminal free amino group, or
b) synthesizing the peptide stepwise,
eliminating one or more protective groups temporarily introduced into the compound obtained as in (a) or (b) to protect other functions where appropriate, and converting the compound of the formula I which has been obtained in this way into its physiologically tolerated salt where appropriate.

3. A peptide of the formula I as claimed in claim 1 for use as medicine.

4. A peptide of the formula I as claimed in claim 1 for the treatment of pathological states which are mediated, induced or assisted by bradykinin and bradykinin-related peptides.

5. A pharmaceutical composition containing a peptide of the formula I as claimed in claim 1.

## Claims (Claims for the following Contracting State(s): ES, GR)

1. A process for the preparation of a peptide of the formula I
H-(D)-Arg-Arg-Pro-Hyp-Gly-E-Ser-(D)-Tic-Oic-Arg-OH (I)
where
E is Leu, Tbg or Cha
and the physiologically tolerated salts thereof,
which comprises
a) reacting a fragment with a C-terminal free carboxyl group or its activated derivative with a corresponding fragment with an N-terminal free amino group, or
b) synthesizing the peptide stepwise,
eliminating one or more protective groups temporarily introduced into the compound obtained as in (a) or (b) to protect other functions where appropriate,
and converting the compound of the formula I which has been obtained in this way into its physiologically tolerated salt where appropriate.

2. A process for the preparation of a pharmaceutical composition containing a peptide of the formula I as claimed in claim 1, which comprises converting the latter and a vehicle into a suitable dosage form.

## Revendications (Revendications pour l'(les) Etat(s) contractant(s) suivant(s): AT, BE, CH, DE, DK, FR, GB, IT, LI, LU, NL, SE)

1. Peptide de formule I
H-(D)-Arg-Arg-Pro-Hyp-Gly-E-Ser-(D)-Tic-Oic-Arg-OH (I)
dans laquelle
E signifie Leu, Tbg ou Cha,
ainsi que ses sels physiologiquement acceptables.

2. Procédé pour la préparation d'un peptide de formule I selon la revendication 1, caractérisé en ce que
a) on fait réagir un fragment avec un groupe carboxyle C-terminal libre ou son dérivé activé, avec un fragment correspondant avec un groupe amino N-termina libre, ou
b) on construit le peptide par étapes,
on élimine éventuellement un ou plusieurs groupes protecteurs introduits temporairement pour protéger d'autres fonctions dans le composé obtenu selon l'étape a) ou b) et on transforme éventuellement le composé de formule I ainsi obtenu en son sel physiologiquement acceptable.

3. Peptide de formule I selon la revendication 1 pour une utilisation comme médicament.

4. Peptide de formule I selon la revendication 1 pour le traitement d'états pathologiques provoqués ou maintenus par la bradykinine et des peptides apparentés à la bradykinine ou dans lequels la bradykinine et les peptides qui lui sont apparentés jouent le rôle de médiateur.

5. Agent pharmaceutique contenant un peptide de formule l selon la revendication 1.

## Revendications (Revendications pour l'(les) Etat(s) contractant(s) suivant(s): ES, GR)

1. Procédé pour la préparation d'un peptide de formule I
H-(D)-Arg-Arg-Pro-Hyp-Gly-E-Ser-(D)-Tic-Oic-Arg-OH (I)
dans laquelle
E signifie Leu, Tbg ou Cha
ainsi que de ses sels physiologiquement acceptables,
caractérisé en ce que :
a) on fait réagir un fragment avec groupe carboxyle C-terminal libre ou son dérivé activé, avec un fragment correspondant à groupe amino N-terminal libre, ou
b) on construit le peptide par étapes,
on élimine éventuellement un ou plusieurs groupes protecteurs introduits temporairement pour protéger d'autres fonctions dans le composé obtenu selon l'étape a) ou b) et
on transforme éventuellement le composé de formule I ainsi obtenu en son sel physiologiquement acceptable.

2. Procédé pour la préparation d'un agent pharmaceutique contenant un peptide de formule I selon la revendication 1, caractérisé en ce que l'on formule sous une forme appropriée à l'administration ledit composé et un véhicule.
